# EUROPEAN PATENT APPLICATION

(11) **EP 2 913 282 A1**
(43) Date of publication of application: **02.09.2015**
(21) Application number: 12886651.4
(22) Date of filing: 19.10.2012
(51) Int. Cl.: B65D 81/18, B65D 33/00, A47J 36/28, B65D 30/10, A61F 7/08

(54) **VERTICAL POUCH**

(71) Applicant: Harvest Charmfoods Co. Ltd., Anyang-si, Gyeonggi-do 431-060 (KR); Krauzen Co., Ltd., Seoul 135-010 (KR)
(72) Inventor: JO, Byung-chul, Seoul 135-230 (KR); CHO, Byung-gu, Seoul 135-904 (KR); CHO, Byung-kwon, Seoul 135-904 (KR)
(74) Representative: Isarpatent
(86) International application number: PCT/KR2012/008617
(87) International publication number: WO 2014/061840

(57) **Abstract**

The present invention relates a vertical pouch including: a heating element to react with a reaction liquid and cause an exothermic reaction; a first pack configured to form a space for containing the heating element between a first sheet and a second sheet; a second pack configured to form a space for containing the heating element between a third sheet and a fourth sheet; a content container comprising the second sheet of the first pack, the third sheet of the second pack, and a bottom connecting lower portions of the second and third sheets and folded upward and downward; and a reaction liquid passage configured to make the first pack communicate with the second pack.

Accordingly, the pouch is minimized in volume while being kept, and is stably stood while being used so that a user can easily eat a content, it is possible to minimize heat transferred to a user's body or the outside when the exothermic reaction occurs, and the heating elements at both sides are all reacted by injecting the reaction liquid into the heating element container at only one side.

## Description

### [TECHNICAL FIELD]

The present invention relates to an vertical pouch.

### [BACKGROUND ART]

To conveniently heat a content without any separate cookware in field or military training, a pouch of a disposable package has been released in the market. A conventional pouch includes an edible content, a content container, a heating element for heating the content, and a reaction liquid inlet for injecting a reaction liquid of reacting with the heating element from the exterior.

The conventional pouch is inconvenient as follows. A user has to vertically hold the pouch to prevent the reaction liquid from flowing out while the reaction liquid is injected into the reaction liquid inlet and makes the heating element generate heat. The user cannot lay the pouch down, but has to hold the pouch with one hand while eating the content with the other hand.

Further, it is uncomfortable for a user to continuously hold the pouch since heat from exothermic reaction is transferred to the user's hand while the user holds the pouch.

In addition, if the heating elements are placed at either side of the content container in the middle of the pouch, it is burdensome for a user to inject the reaction liquid into both sides.

Generally, left and right heating element containers are arranged to surround the content container in the middle, and the content container in the middle is heated by heat generated by the reaction of the heating elements. In this case, problems of heat loss and inconvenience arise since a considerable amount of heat leaks through the outer sheets of the heating element containers.

### [DISCLOSURE]

### [TECHNICAL PROBLEM]

An aspect of the invention is to provide a vertical pouch which can be stably stood up.

Another aspect of the invention is to provide a vertical pouch which can minimize heat transferred to a user's body or the outside.

Still another aspect of the invention is to provide a vertical pouch in which heating elements at either side are all reacted by injecting a reaction liquid into a heating element container at one side.

### [TECHNICAL SOLUTION].

The foregoing and/or other aspects of the present invention are achieved by providing a vertical pouch including: a heating element to react with a reaction liquid and cause an exothermic reaction; a first pack configured to form a space for containing the heating element between a first sheet and a second sheet; a second pack configured to form a space for containing the heating element between a third sheet and a fourth sheet; a content container comprising the second sheet of the first pack, the third sheet of the second pack, and a bottom connecting lower portions of the second and third sheets and folded upward and downward; and a reaction liquid passage configured to make the first pack communicate with the second pack.

The reaction liquid passage may be formed by penetrating an adhered portion where the second sheet and the third sheet are adhered, or by bending and adhering edge portions of the second and third sheets from edges of the first and fourth sheets.

The reaction liquid passage may be formed higher than a fold when the bottom is folded upward.

The first sheet, the second sheet, the third sheet and the fourth sheet may include a plurality of sheet members, an air-gap may be formed between the sheet members, and at least one of the sheet members may include an insulating sheet member.

At least one of the first sheet and the fourth sheet further may include a reaction liquid inlet through which a reaction liquid can be injected, and the first pack and the second pack may further include a reaction liquid pack to accommodate a reaction liquid.

### [ADVANTAGEOUS EFFECTS]

According to the vertical pouch configured as described above has at least one of the following effects.

First, the pouch is minimized in volume while being kept, and is stably stood while being used so that a user can easily eat a content.

Second, it is possible to minimize heat transferred to a user's body or the outside when the exothermic reaction occurs.

Third, the heating elements at both sides are all reacted by injecting the reaction liquid into the heating element container at only one side.

### [DESCRIPTION OF DRAWINGS]

FIG. 1 is a perspective view of a vertical pouch according a first embodiment of the present invention,
FIG. 2 is a sectional view of the vertical pouch according to the first embodiment of the present invention,
FIG. 3 is an enlarged sectional view of the vertical pouch according to the first embodiment of the present invention,
FIG. 4 is a perspective view of a vertical pouch according to a second embodiment of the present invention,
FIG. 5 is an enlarged sectional view of the vertical pouch according to the second embodiment of the present invention,
FIG. 6 is a sectional view of a vertical pouch according to a third embodiment of the present invention, and
FIG. 7 is a perspective view of a vertical pouch according to a fourth embodiment of the present invention.

### [BEST MODE]

Below, embodiments of the present invention will be described with reference to accompanying drawings.

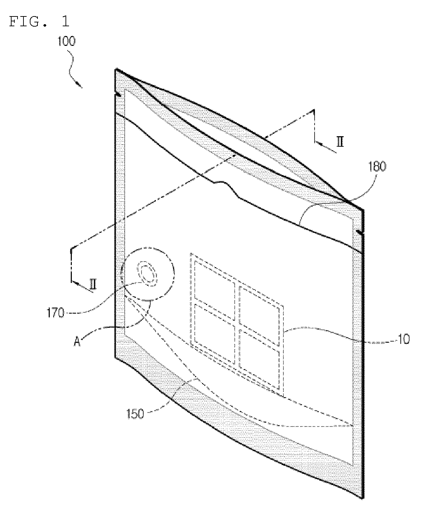

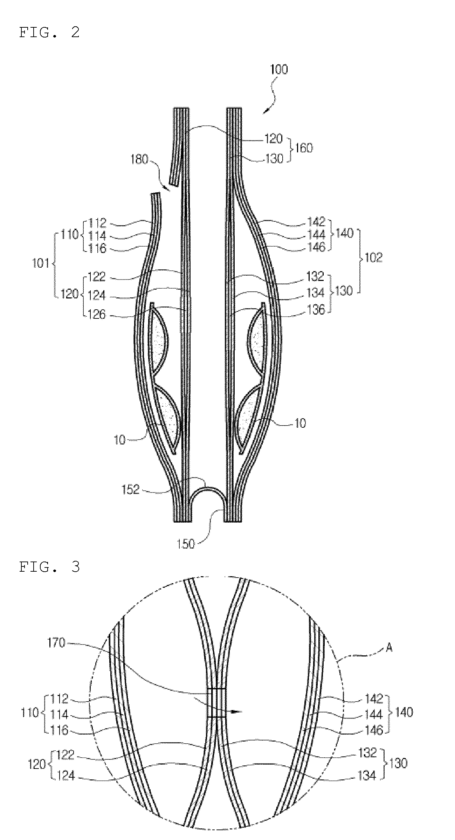

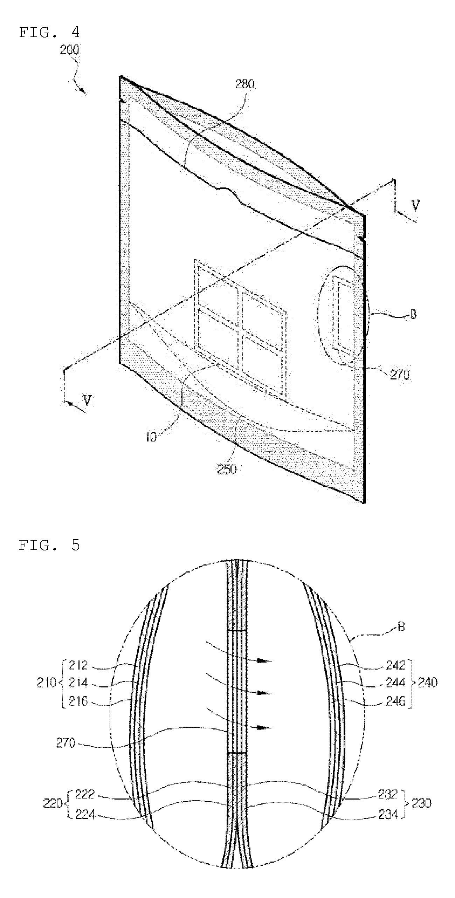

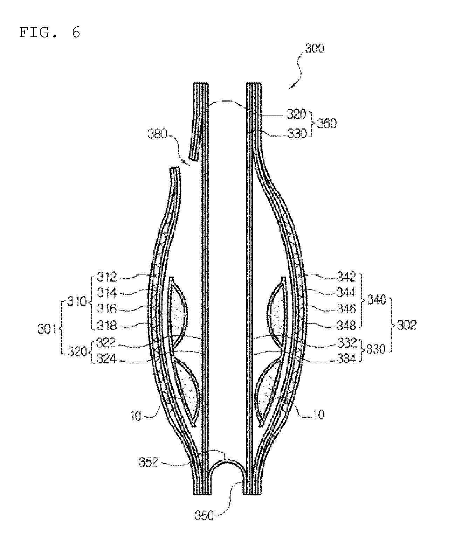

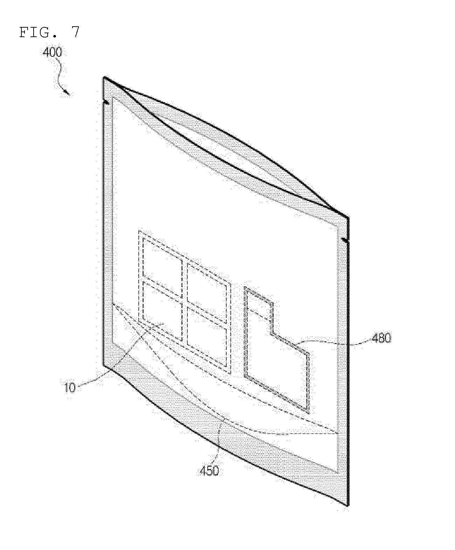

FIG. 1 is a perspective view of a pouch 100 according a first embodiment of the present invention, FIG. 2 is a sectional view of the pouch 100 according to the first embodiment of the present invention, FIG. 3 is an enlarged sectional view of a reaction liquid passage 170 according to the first embodiment of the present invention, FIG. 4 is a perspective view of a pouch 200 according to a second embodiment of the present invention, FIG. 5 is an enlarged sectional view of a reaction liquid passage 270 in the pouch 200 according to the second embodiment of the present invention, FIG. 6 is a sectional view showing that a first sheet 310 and a fourth sheet 340 of a pouch 300 include air-gaps 318 and 348 according to a third embodiment of the present invention, and FIG. 7 is a perspective view of a pouch 400 with a reaction liquid pack 480 according to a fourth embodiment of the present invention.

As shown in FIGs. 1 and 2, the vertical pouch 100 according to the first embodiment of the present invention includes a first pack 101, a second pack 102, and a content container 160 formed between the first pack 101 and the second pack 102.

In the first pack 101, a first sheet 110 and a second sheet 120 are adhered to each other only at its ending part so as to form a space in the middle of the first sheet 100 and the second sheet 120. A heating element 10 is accommodated in the middle space formed by adhering the first sheet 110 and the second sheet 120.

The heating element 10 may include a liquid or solid material that reacts with a reaction liquid and causes an exothermic reaction. Preferably, the sold material may be used to prevent the heating element 10 from leaking out. The solid heating element 10 may include one or more among calcium oxide (CaO), calcium hydroxide (Ca(OH)₂), magnesium chloride (MgCl₂), iron (Fe) and aluminum (Al).

The adhering process may be achieved by a method of piling up the first sheet 110, the heating element 10 and the second sheet 120 in sequence and adhering four edges thereof, or a method of first adhering the edges of the first sheet 110 and the second sheet 120 in the form of 'U' except the upper edge and then sealing up the upper edge after inserting the heating element 10 through the upper edge.

The second pack 102 may be formed by adhering edges of a third sheet 130 and a fourth sheet 140, and accommodates the heating element 10 in a space formed by adhering the third sheet 130 and the fourth sheet 140 like the first pack 101. Such adhesion between the sheets 110, 120, 130 and 140 may be achieved by heat sealing, high frequency sealing or the like.

As shown in FIG. 2, the first sheet 110 forming an outer surface of the pouch 100 includes a plurality of sheet members 112, 114 and 116. Like the first sheet 110, the fourth sheet 140 also includes a plurality of sheet members 142, 144 and 146.

When heat is generated inside the pouch 100, heat is transferred to a user's body being in contact with the pouch 100 or to the outside. To minimize the transfer of the heat, the first sheet 110 and the fourth sheet 140 include a plurality of layers. As the first sheet 110 and the fourth sheet 140 are formed by the plurality of layers, it is also possible to protect the heating element 10 or the content from external shock. If a reaction liquid bag is enveloped together with the heating element 10, the plurality of layers may prevent the reaction liquid bag from being damaged by external shock and wastefully react with the heating element 10.

Sheet members 112 and 142 provided in the outmost surface of the first sheet 110 and the fourth sheet 140 are comprised of by insulating sheet members so as to further minimize heat transfer to a user. As necessary, the insulating sheet member may be applied to not only the outmost sheet members 112 and 142 but also the other sheet members 114, 116, 144 and 146, and a plurality of insulating sheet members may be used for the first sheet 110 and the fourth sheet 140. The insulating sheet member may include polyurethane, polystyrene, calcium silicate or the like.

Like the first sheet 110 and the fourth sheet 140, the second sheet 120 of the first pack 101 and the third sheet 130 of the second pack 102 may include a plurality of sheet members. For example, the second sheet 120 may include two sheet members 122 and 124 and an air-gap 126 formed between the plurality of sheet members 122 and 124. Like the second sheet 120, the third sheet 130 may include two sheet members 132 and 134 and an air-gap 136 between the sheet members 132 and 134.

If the second sheet 120 and the third sheet 130 are each comprised of a single sheet, the heating element 10, reaction gas generated by reaction between the reaction liquid and the heating element 10, or the like may flow in the content container 160 when a minute pinhole is generated in the second sheet 120 and the third sheet 130. In order to prevent this, the second sheet 120 and the third sheet 130 are configured to the plurality of sheet members 122, 124, 132 and 134, respectively.

The air-gaps 126 and 136 serve to protect the content accommodated in the content container 160 from being damaged or deformed by external shock. The air-gap 126 is formed by adhering edges of the sheet members 122 and 124 to form a space in the middle. This manner is also applied to the air-gap 136 of the third sheet 130.

The second sheet 120 and the third sheet 130 are adhered to each other at opposite longitudinal end portions. The bottom 150 is adhered to the lower portions of the second sheet 120 and the third sheet 130. The bottom 150 includes a fold 152 to be folded upward or downward in the pouch 100.

The bottom 150 is folded usually, and is pulled and stretched out in directions of the first sheet 110 and the fourth sheet 140 and laid down so that the pouch 100 can be stood. When the bottom 150 is folded, the pouch 100 is decreased in volume to be easily kept. When the bottom 150 is stretched out, the pouch 100 can be stood to be convenient for a user to eat the content in the pouch 100. Further, if the pouch 100 is stood during the exothermic reaction of the heating element 10, a user is free from getting burned since the user does not have to hold the pouch 100 with the user's hand.

Thus, the content container 160 formed by the second sheet 120, the third sheet 130 and the bottom 150 is provided inside the pouch 100. The content container 160 may be filled with the content such as food, and the content such as food is heated by the heating element 10. If food is directly filled in the content container 160, the second sheet 120, the third sheet 130 and the bottom 150 may be made of materials harmless to humans.

The first pack 101 and the second pack 102 include a reaction liquid passage 170 through which they communicate with each other. As shown in the enlarged sectional view of FIG. 3, the reaction liquid passage 170 is formed to make the first pack 101 and the second pack 102 communicate with each other so that the reaction liquid introduced into the first pack 101 can flow to the second pack 102. Alternatively, the reaction liquid introduced into the second pack 102 may flow to the first pack 101. According to the first embodiment, the reaction liquid passage 170 is formed by penetrating an adhered portion where the second sheet 120 and the third sheet 130 are partially adhered. The adhered portion has to be penetrated so that the reaction liquid can flow only to the second pack 102 without leaking to the content container 160.

The reaction liquid passage 160 may be formed higher than the fold when the bottom 150 is folded upward. If the reaction liquid passage 160 is placed below the fold 152, the bottom 150 is not completely stretched out. The pouch 100 can be stood only when the bottom 150 has to be stretched out as much as a predetermined distance. However, if the reaction liquid passage 170 is placed below the fold 152, i.e. if the second sheet 120 and the third sheet 130 are adhered close by the bottom 150, this interferes with stretching out the bottom 150. Thus, the reaction liquid passage 160 is preferably formed above the fold 152 so as not to interfere with the bottom 150 that is largely changed in position.

At least one of the first sheet 110 and the fourth sheet 140 may include a reaction liquid inlet 180 through which the reaction liquid can be injected. If the reaction liquid inlet 180 is formed in the first sheet 110, the reaction liquid is injected into the first pack 101. If the reaction liquid inlet 180 is formed in the fourth sheet 140, the reaction liquid is injected into the second pack 102. Then, the injected reaction liquid moves to the other pack via the reaction liquid passage 170. Since the reaction liquid is injected through the reaction liquid inlet 180, the reaction liquid passage 170 is preferably placed below the reaction liquid inlet 180 so that the reaction liquid can flow to the other pack. Alternatively, the reaction liquid inlet 180 may be formed in both the first sheet 110 and the fourth sheet 140 so that the reaction liquid can be injected at both sides.

As shown in FIGs. 4 to 5, a vertical pouch 200 according to a second embodiment of the present invention includes a first pack (not shown), a second pack (not shown) and a bottom 250 formed between the first and second packs. A first sheet 210 and a fourth sheet 240 include a plurality of sheet members 212, 214, 216, 242, 244 and 246, and the outmost sheet members 212 and 242 may be an insulating sheet member. Further, a reaction liquid inlet 280 is formed in the first sheet 210, the fourth sheet 240 or both the first sheet 210 and the fourth sheet 240. Likewise, a second sheet 220 and a third sheet 230 include a plurality of sheet members 222, 224, 232 and 234.

The elements of the second embodiment are similar to those of the first embodiment. However, the first and second embodiments are different in shape of a reaction liquid passage 270 formed between a second sheet 220 and a third sheet 230.

As shown in FIG. 4, the portion of the second sheet 220 and the third sheet 230 are grooved in the form of 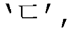 and this portion is differentiated in that only the second sheet 220 and the third sheet 230 are adhered to each other. The first sheet 210, the second sheet 220, the third sheet 230 and the fourth sheet 240 are all adhered at end portions in a transverse direction except the portion in the form of 'U'. But the second sheet 220 is adhered to the third sheet 230 and the first sheet 210 is adhered to the fourth sheet 240 at the portion grooved in the form of 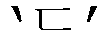 as shown in FIG. 4 so that the portion formed by the adhesion can function as a reaction liquid passage 270 through which the reaction liquid passes. Alternatively, the reaction liquid passage 270 may vary in area, shape and position.

The reaction liquid passage 270 shown in FIG. 5 serves to make the reaction liquid flow from the first pack 201 to the second pack 202 or from the second pack 202 to the first pack 201 like that of the first embodiment. On the contrary to the first embodiment, the reaction liquid passage 270 is formed in the end portion of the pouch 200 so that the content can be filled in the pouch 200 without being interfered with the reaction liquid passage 270. The reaction liquid passage 170 of the first embodiment and the reaction liquid passage 270 of the second embodiment are similar in their function and shape, but different in the manufacturing process.

According to the third embodiment, the pouch 300 includes a first pack 301, a second pack 302, and a bottom 350 formed between the first pack 301 and the second pack 302, and includes a content container 360 in the middle. The first sheet 310 and the fourth sheet 340 include a plurality of sheet members 312, 314, 316, 342, 344 and 346, and the outmost sheet members 312 and 342 may be an insulating sheet member. Further, a reaction liquid inlet 280 may be formed in the first sheet 310, the fourth sheet 340 or both the first sheet 310 and the fourth sheet 340.

The bottom 350 includes a fold 352 so that the bottom 350 can be folded upward.

In addition, the second sheet 320 and the third sheet 330 include a plurality of sheet members 322, 324, 332 and 334.

The elements of the third embodiment are similar to those of the second embodiment, but different in some portions of the first sheet 310 and the fourth sheet 340.

As shown in FIG. 6, the first sheet 310 and the fourth sheet 340 include the plurality of sheet members 312,314, 316, 342, 344 and 346 in order to minimize heat transferred to a user's body. In particular, the outmost sheet members 312 and 342 are made of insulating sheet members to further minimize heat transfer.

In addition, an air-gap 318 is formed in between the outmost sheet member 312 and the second sheet member 314 of the first sheet 310, so that the heat transfer can be more minimized than that of when only the plurality of sheet members 312, 314 and 316 constitutes the first sheet 310. Alternatively, the air-gap 318 may be formed in between the second sheet member 314 and the third sheet member 316. Like the first sheet 310, an air-gap 348 may be added in between the sheet members 342, 344 and 346 of the fourth sheet 340 to minimize heat transferred to a user.

The air-gaps 318 and 348 may use an air cap which is generally used for protecting a product and in which a plurality of air bubbles are arranged. Besides, the sheet members 312, 314, 316, 342, 344 and 346 of the first sheet 310 and the fourth sheet 340 may be adhered in the form of lattice at edges and insides, and air may be injected in the lattices not adhered between the sheet members 312, 314, 316, 342, 344 and 346.

According to the fourth embodiment, the pouch 400 includes the first pack 101, the second pack 102, and a bottom 450 adhered to the lower portions of the first pack 101 and the second pack 102 and forming the content container 160.

The first pack 101 includes the first sheet 110 and the second sheet 120, and the second pack 102 is formed by adhering only the edges of the third sheet 130 and the fourth sheet 140.

The heating element 10 is added in between the first pack 101 and the second pack 102.

The first sheet 110 and the fourth sheet 140 include the plurality of sheet members 112, 114, 116, 142, 144 and 146, and the outmost sheets 112 and 142 may be an insulating sheet member. Further, the second sheet 120 and the third sheet 130 also include the plurality of sheet members 122, 124, 132 and 134.

These elements are the same as those of the first embodiment, but the fourth embodiment does not include the reaction liquid inlet 180.

As shown in FIG. 7, the pouch 400 does not include the reaction liquid inlet 180 but instead includes a reaction liquid pack 480 placed in one space, where the heating element 10 is placed, between the first sheet 110 and the second sheet 120 or between the third sheet 130 and the fourth sheet 140.

In the case where heat generation is caused from the outside of the pouch, it may be impossible to get the reaction liquid, i.e. water. In this case, the pouch 400 provided with the reaction liquid pack 480 is used so that the pouch 400 can be easily heated.

The reaction liquid pack 480 may be formed to be torn open by predetermined force from the exterior so that the heating element 10 and the reaction liquid can meet, or the reaction liquid pack 480 may have a tearing member and be torn by the tearing member so that the reaction liquid can flow from the reaction liquid pack 480.

According to the foregoing embodiments, the vertical pouch 100, 200, 300, 400 can be vertically laid on a table or the like by stretching out the folded bottom 150, 250, 350, 450 if it is inconvenient for a user to hold the pouch 100, 200, 300, 400 with one hand and take the content out with the other hand, thereby allowing him/her to easily take the content.

Further, the first sheet 110, 210, 310 and the fourth sheet 140, 240, 340 include the plurality of layers so that heat transferred to a user's body can be minimized when the exothermic reaction occurs, and the insulating sheet member included in the plurality of layers include the air-gap 318, 348 so that a user can hold the pouch 100, 200, 300, 400 without inconvenience.

In addition, the second sheet 120, 220, 320 and the third sheet 130, 230, 330 include the plurality of layers so that the heating element 10, the reaction liquid or reaction gas caused by the reaction between them cannot be introduced into the content container 160.

Furthermore, if the heating elements 10 are separately provided at both sides of the pouch 100, 200, 300, 400, to the reaction liquid has to be conventionally put into both sides. However, the pouch 100, 200, 300, 400 according to the present invention includes the reaction liquid passage 170, 270 so that the reaction liquid injected into one of the two sides can be enough to make all the heating elements 10 generate heat. Accordingly, it is convenient for a user.

## Claims

1. A vertical pouch comprising:
a heating element to react with a reaction liquid and cause an exothermic reaction;
a first pack configured to form a space for containing the heating element between a first sheet and a second sheet;
a second pack configured to form a space for containing the heating element between a third sheet and a fourth sheet;
a content container comprising the second sheet of the first pack, the third sheet of the second pack, and a bottom connecting lower portions of the second and third sheets and folded upward and downward; and
a reaction liquid passage configured to make the first pack communicate with the second pack.

2. The vertical pouch according to claim 1, wherein the reaction liquid passage is formed by penetrating an adhered portion where the second sheet and the third sheet are adhered.

3. The vertical pouch according to claim 1, wherein the reaction liquid passage is formed by bending and adhering edge portions of the second and third sheets from edges of the first and fourth sheets.

4. The vertical pouch according to any one of claims 1 and 2, wherein the reaction liquid passage is formed higher than a fold when the bottom is folded upward.

5. The vertical pouch according to claim 1, wherein the first sheet, the second sheet, the third sheet and the fourth sheet comprise a plurality of sheet members.

6. The vertical pouch according to claim 5, further comprising an air-gap between the sheet members.

7. The vertical pouch according to claim 5, wherein at least one of the sheet members comprises an insulating sheet member.

8. The vertical pouch according to claim 1, wherein at least one of the first sheet and the fourth sheet further comprises a reaction liquid inlet through which a reaction liquid can be injected.

9. The vertical pouch according to claim 1, wherein the first pack and the second pack further comprise a reaction liquid pack to accommodate a reaction liquid.
